# EUROPÄISCHE PATENTSCHRIFT

(11) **EP 2 931 413 B1**
(45) Veröffentlichungstag und Bekanntmachung des Hinweises auf die Patenterteilung: **09.08.2017**
(21) Anmeldenummer: 13802644.8
(22) Anmeldetag: 11.12.2013
(51) Int. Cl.: B01J 8/04, B01J 19/24, C07C 5/48

(54) **REAKTOR ZUR DURCHFÜHRUNG EINER AUTOTHERMEN GASPHASENDEHYDRIERUNG**
REACTOR FOR PERFORMING AN AUTOTHERMAL GAS-PHASE DEHYDROGENATION
RÉACTEUR POUR LA RÉALISATION D'UNE DÉSHYDROGÉNATION AUTOTHERMIQUE EN PHASE GAZEUSE

(30) Priorität: 12.12.2012 EP 12196666
(43) Veröffentlichungstag der Anmeldung: 21.10.2015
(73) Patentinhaber: BASF SE, 67056 Ludwigshafen am Rhein (DE)
(72) Erfinder: OLBERT, Gerhard, 69221 Dossenheim (DE); TELLAECHE HERRANZ, Carlos, 69126 Heidelberg (DE); ASPRION, Norbert, 67063Ludwigshafen (DE); WECK, Alexander, 67251 Freinsheim (DE); DAHLHOFF, Ellen, 67117 Limburgerhof (DE)
(86) Internationale Anmeldenummer: PCT/EP2013/076154
(87) Internationale Veröffentlichungsnummer: WO 2014/090841

(56) Entgegenhaltungen:
- EP-A2- 0 568 303
- WO-A1-2012/084609
- US-A- 3 475 136
- US-A1- 2012 157 737

## Beschreibung

Die Erfindung betrifft einen Reaktor zur Durchführung von autothermen Gasphasendehydrierungen unter Verwendung eines heterogenen Katalysators, der als Monolith ausgebildet ist sowie ein Verfahren unter Verwendung des Reaktors.

Keramische oder metallische Monolithe sind als Katalysatorträger für Edelmetallkatalysatoren in der mobilen und stationären Abgasreinigung etabliert. Die Kanäle bieten der Strömung einen geringen Strömungswiderstand bei gleichzeitig gleichmäßiger Zugänglichkeit der äußeren Katalysatoroberfläche für gasförmige Reaktionsmedien. Dies ist vorteilhaft gegenüber regellosen Haufwerken, bei denen durch unzählige Umlenkungen bei der Strömung um die Partikel ein großer Druckverlust entsteht und die Katalysatoroberfläche eventuell nicht gleichmäßig genutzt wird. Der Einsatz von Monolithen ist generell interessant für katalytische Prozesse mit hohen Volumenströmen und adiabater Reaktionsführung bei hohen Temperaturen. Diese Merkmale treffen in der chemischen Produktionstechnik insbesondere für Dehydrierungsreaktionen zu, die in einem Temperaturbereich von 400 °C bis zu 700 °C ablaufen.

Fortschritte in der Katalysatortechnik ermöglichen die selektive Verbrennung des Dehydrierwasserstoffes in Anwesenheit von Kohlenwasserstoffen, wie beispielsweise in US 7,034,195 beschrieben. Eine derartige Fahrweise wird als autotherme Dehydrierung bezeichnet und erlaubt, Dehydrierreaktoren direkt zu beheizen, so dass aufwändige Vorrichtungen zur indirekten Vor- und Zwischenheizung des Reaktionsgemisches entfallen. Ein derartiges Verfahren ist beispielsweise in US 2008/0119673 beschrieben. Dieses Verfahren besitzt jedoch den gravierenden Nachteil, dass die Dehydrierung an einem heterogenen Katalysator in Pelletform durchgeführt wird: Der hohe Strömungswiderstand von Pelletschüttungen erfordert einen großen Reaktorquerschnitt und eine entsprechend niedrige Durchströmungsgeschwindigkeit, um den Druckabfall in der katalytisch aktiven Schicht zu begrenzen. Dieser Nachteil wird durch eine sehr aufwändige Vorrichtung zur Dosierung und Verteilung des Sauerstoffes ausgeglichen, was den Vorteil der autothermen Dehydrierung beeinträchtigt.

Die EP-A 2 506 963 stellt einen Reaktor in Form eines liegenden Zylinders zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches an einem heterogenen Katalysator, der als Monolith ausgebildet ist, zur Verfügung, wobei
- der Innenraum des Reaktors durch ein lösbar, in Längsrichtung des Reaktors angeordnetes kreiszylindrisches oder prismatisches, in Umfangsrichtung gasdichtes, an beiden Stirnseiten desselben offenes Gehäuse G in
- einen Innenbereich A, mit einer oder mehreren katalytisch aktiven Zonen, worin jeweils eine Packung aus aufeinander, nebeneinander und hintereinander gestapelten Monolithen und vor jeder katalytisch aktiven Zone jeweils eine Mischzone mit festen Einbauten vorgesehen ist und
- einen koaxial zum Innenbereich A angeordneten Außenbereich B aufgeteilt ist,
- mit einer oder mehreren Zuführleitungen für den zu dehydrierenden kohlenwasserstoffhaltigen Gasstrom in den Außenbereich B, Umlenkung des zu dehydrierenden Kohlenwasserstoffstroms an einem Ende des Reaktors und Zuführung über einen Strömungsgleichrichter in den Innenbereich A,
- mit einer oder mehreren, unabhängig voneinander regelbaren Zuführleitungen, wobei jede Zuführleitung eine oder mehrere Verteilerkammern versorgt, für den Sauerstoff enthaltenden Gasstrom in jede der Mischzonen sowie
- mit einer Abführleitung für das Reaktionsgemisch der autothermen Gasphasendehydrierung am Ende des Reaktors wie die Zuführleitung für den zu dehydrierenden Kohlenwasserstoffstrom.

An dem Reaktorende, an dem die Abführleitung für das Reaktionsgasgemisch der autothermen Gasphasendehydrierung angeordnet ist, ist vorteilhaft ein Rohrbündelwärmetauscher vorgesehen, mit einem Bündel von Rohren, durch die das Reaktionsgasgemisch zur autothermen Gasphasendehydrierung geleitet wird, sowie mit Zwischenräumen zwischen den Rohren, durch die, im Gegenstrom zum Reaktionsgemisch der autothermen Gasphasendehydrierung, der zu dehydrierende kohlenwasserstoffhaltige Gasstrom geleitet wird.

Die WO 2012/084609 schlägt, ausgehend von der EP-A 2 506 963 einen aus sicherheitstechnischer Sicht verbesserten Reaktor vor, wonach der Außenbereich B mit einem unter den Reaktionsbedingungen der autothermen Gasphasendehydrierung inerten Gas beaufschlagt ist und dass der zu dehydrierende kohlenwasserstoffhaltige Gasstrom über eine Zuführleitung in den Wärmetauscher eingeleitet, durch das Reaktionsgasgemisch im Gegenstrom durch indirekten Wärmetausch aufgeheizt und weiter an das dem Wärmetauscher entgegengesetzte Ende des Reaktors geleitet, dort umgelenkt, über einen Strömungsgleichrichter in den Innenbereich A eingeleitet, und in den Mischzonen mit dem Sauerstoff enthaltenden Gasstrom vermischt wird, worauf im Innenbereich A des Reaktors die autotherme Gasphasendehydrierung stattfindet.

Das Design der obigen Reaktoren ist jedoch aufwändig, insbesondere durch die Aufteilung des Reaktorinnenraumes in einen Innenbereich und einen Außenbereich, in dem ein in Längsrichtung des Reaktors angeordnetes Gehäuse vorgesehen ist.

Es war demgegenüber Aufgabe der Erfindung, einen Reaktor zur Durchführung von autothermen Gasphasendehydrierungen unter Verwendung eines heterogenen Katalysators, der als Monolith ausgebildet ist, zur Verfügung zu stellen, der ein demgegenüber deutlich einfacheres Design aufweist und der einen einfachen Austausch der Monolithe, nach Bedarf, gewährleistet.

Die Aufgabe wird gelöst durch einen Reaktor in Form eines Zylinders mit vertikaler Längsachse zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches, an einem heterogenen Katalysator, der als Monolith ausgebildet ist, wobei
- im Innenraum des Reaktors eine oder mehrere katalytisch aktive Zonen angeordnet sind, umfassend jeweils eine Packung aus neben- und/oder übereinander gestapelten Monolithen und wobei vor jeder katalytisch aktiven Zone jeweils eine Mischzone mit festen Einbauten vorgesehen ist,
- mit einer oder mehreren Zuführleitungen für den zu dehydrierenden kohlenwasserstoffhaltigen Gasstrom am unteren Ende des Reaktors,
- mit einer oder mehreren, unabhängig voneinander regelbaren Zuführleitungen, wobei jede Zuführleitung einen oder mehrere Verteiler versorgt, für den Sauerstoff enthaltenden Gasstrom in jede der Mischzonen sowie
- mit einer oder mehreren Abführleitungen für das Reaktionsgasgemisch der autothermen Gasphasendehydrierung am oberen Ende des Reaktors
wobei die Innenwand des Reaktors durchgehend mit einer Isolationsschicht versehen ist und wobei die Zugänglichkeit der einen oder jeder der mehreren katalytisch aktiven Zonen von außerhalb des Reaktors über
- jeweils ein oder mehrere Mannlöcher gewährleistet ist oder
- wobei die eine oder jede der mehreren katalytisch aktiven Zonen, umfassend jeweils eine Packung aus neben- und/oder übereinander gestapelten Monolithen einschließlich
   - der vor jeder katalytisch aktiven Zone vorgesehenen Mischzone mit festen Einbauten,
   - der einen oder mehreren, unabhängig voneinander regelbaren Zuführleitungen und
   der einen oder mehreren Verteiler, die von jeweils einer Zuführleitung versorgt werden, jeweils als ein Bauelement ausgebildet ist, das einzeln montier- und demontierbar ist.

Die einzelnen Bauelemente können beispielsweise über Schweißnähte zusammengefügt und auseinandergetrennt werden.

Insbesondere können die einzelnen Bauelemente mittels Flanschen zusammengefügt und auseinandergetrennt werden.

In einer Ausführungsform wird die Aufgabe gelöst durch einen Reaktor in Form eines Zylinders mit vertikaler Längsachse zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches, an einem heterogenen Katalysator, der als Monolith ausgebildet ist, wobei
- im Innenraum des Reaktors eine oder mehrere katalytisch aktive Zonen angeordnet sind, umfassend jeweils eine Packung aus neben- und/oder übereinander gestapelten Monolithen und wobei vor jeder katalytisch aktiven Zone jeweils eine Mischzone mit festen Einbauten vorgesehen ist,
- mit einer oder mehreren Zuführleitungen für den zu dehydrierenden kohlenwasserstoffhaltigen Gasstrom am unteren Ende des Reaktors,
- mit einer oder mehreren, unabhängig voneinander regelbaren Zuführleitungen, wobei jede Zuführleitung einen oder mehrere Verteiler versorgt, für den Sauerstoff enthaltenden Gasstrom in jede der Mischzonen sowie
- mit einer oder mehreren Abführleitungen für das Reaktionsgasgemisch der autothermen Gasphasendehydrierung am oberen Ende des Reaktors
wobei die eine oder jede der mehreren katalytisch aktiven Zonen über jeweils ein oder mehrere Mannlöcher von außerhalb des Reaktors zugänglich ist,
und wobei die Innenwand des Reaktors durchgehend mit einer Isolationsschicht versehen ist.

Erfindungsgemäß wird somit ein Reaktor vorgeschlagen, der in Form eines Zylinders mit vertikaler Längsachse ausgebildet ist, d.h. als aufrecht stehender Apparat.

Die Monolithe sind in den katalytisch aktiven Zonen der Gestalt eingebaut, dass die Kanäle der Monolithe in vertikaler Richtung durchströmt werden.

Die autotherme Gasphasendehydrierung findet an einem heterogenen Katalysator statt, der in Form von Monolithen vorliegt.

Als Monolith wird vorliegend ein einstückiger, parallelepipedischer Block mit einer Vielzahl von parallel zueinander angeordneten, durchgehenden Kanälen mit engem Querschnitt, im Bereich von etwa 0,36 bis 9 mm², verstanden. Die Kanäle sind bevorzugt mit quadratischem Querschnitt ausgebildet, insbesondere mit einer Seitenlänge des Quadrates im Bereich von 0,6 bis 3 mm, besonders bevorzugt von 1,0 bis 1,5 mm.

Die Monolithe sind bevorzugt aus einem keramischen Werkstoff als Trägermaterial gebildet, worauf eine katalytisch aktive Schicht, bevorzugt nach dem sogenannten Wash-Coating-Verfahren, aufgebracht ist.

Das gängigste Material für monolithische Strukturen ist Cordierit (ein Keramikmaterial, das aus Magnesiumoxid, Siliciumoxid und Aluminiumoxid im Verhältnis 2:5:2 besteht). Andere Materialien, deren Monolithstrukturen im Handel erhältlich sind, sind Metalle, Mullit (Mischoxid von Siliciumoxid und Aluminiumoxid, Verhältnis 2:3) und Siliciumcarbid. Diese Materialien haben ähnlich wie Cordierit eine niedrige spezifische BET-Oberfläche (BET = Brunauer, Emmet und Teller) (z.B. für Cordierit typischerweise 0,7 m²/g).

Monolithische Keramikelemente sind mit Zelldichten von 25 - 1600 cpsi (Zellen pro Quadratzoll, entspricht einer Zellgröße von 5 - 0,6 mm) erhältlich. Durch Verwendung einer höheren Zelldichte nimmt die geometrische Oberfläche zu, so dass der Katalysator effizienter verwendet werden kann. Nachteile von höheren Zelldichten sind ein etwas schwierigeres Herstellungsverfahren, eine schwierigere Washcoat-Beschichtung und ein höherer Druckverlust über den Reaktor. Des Weiteren sind in der Regel bei großen Zelldichten auch die Stege dünner, was die mechanische Stabilität der Monolithe verringert. In zylindrischen Reaktoren sind die Monolithe im Randbereich durch entsprechenden Zuschnitt anzupassen. Der Druckverlust bleibt jedoch für Monolithen mit hoher Zelldichte im Vergleich zu einem Füllkörperreaktor sehr gering (in der Regel um den Faktor 10 geringer), was auf die geraden Monolithkanäle zurückzuführen ist.

Zur Herstellung von monolithischen Keramikelementen kann man eine Mischung von Talk, Ton und einer aluminiumoxidliefernden Komponenten und Siliciumdioxid herstellen, die Mischung zur Bildung einer Formmasse mischen, die Mischung formen, die Rohware trocknen und sie bei einer Temperatur von 1200 bis 1500 °C erhitzen, wobei man eine Keramik erhält, die hauptsächlich Cordierit enthält und einen niedrigen Wärmeausdehnungskoeffizienten aufweist. Allgemein gesprochen kann man eine Paste mit entsprechenden rheologischen Eigenschaften und entsprechender rheologischer Zusammensetzung zu einem Monolithträger extrudieren. Die Paste besteht in der Regel aus einer Mischung von Keramikpulvern geeigneter Größe, anorganischen und/oder organischen Additiven, Lösungsmittel (Wasser), Peptisierungsmittel (Säure) zur Einstellung des pH-Werts und einem permanenten Bindemittel (kolloidale Lösung oder Sol). Bei den Additiven kann es sich um einen Weichmacher oder ein Tensid zur Einstellung der Viskosität der Paste oder ein temporäres Bindemittel, das später abgebrannt werden kann, handeln. Zuweilen werden Glas- oder Kohlefasern zur Erhöhung der mechanischen Festigkeit des Monolithen zugesetzt. Das permanente Bindemittel sollte die innere Festigkeit des Monolithen verbessern.

Cordierit-Monolithe können aus einer Charge hergestellt werden, die aus Talk, Kaolin, calciniertem Kaolin und Aluminiumoxid besteht und zusammen eine chemische Verbindung aus 45 bis 55 Gew.-% SiO₂, 32 bis 40 Gew.-% Al₂O₃ und 12 bis 15 Gew.-% MgO liefern. Talk ist ein Material, das hauptsächlich aus Magnesiumsilicathydrat, Mg₃Si₄O₁₀(OH)₂ besteht. Der Talk kann je nach Quelle und Reinheit auch mit anderen Mineralien wie Tremolit (CaMg₃(SiO₃)₄), Serpentin (3MgO.2SiO₂, 2H₂O), Anthophyllit (Mg₇(OH)₂(Si₄O₁₁)₂), Magnesit (MgCO₃), Glimmer und Chlorit vergesellschaftet sein.

Durch Extrusion können auch Monolithe aus anderen Materialien wie SiC, B₄C, Si₃N₄, BN, AIN, Al₂O₃, ZrO₂, Mullit, Al-Titanat, ZrB₂, Sialon, Perowskit, Kohlenstoff und TiO₂ hergestellt werden.

Von Bedeutung hinsichtlich der Eigenschaften der Monolithprodukte sind bei der Extrusion neben der Qualität der Düse, der Art und den Eigenschaften der zur Herstellung der formbaren Mischung verwendeten Materialien auch die zugesetzten Additive, der pH-Wert, der Wassergehalt und die bei der Extrusion verwendete Kraft. Bei den bei der Extrusion angewandten Additiven handelt es sich beispielsweise um Cellulosen, CaCl₂, Ethylenglykole, Diethylenglykole, Alkohole, Wachs, Paraffin, Säuren und hitzebeständige anorganische Fasern. Neben Wasser können auch andere Lösungsmittel verwendet werden, wie Ketone, Alkohole und Ether. Der Zusatz von Additiven kann zu verbesserten Eigenschaften der Monolithe, wie der Bildung von Mikrorissen, die die Temperaturwechselbeständigkeit verbessert, besserer Porosität und besserem Absorptionsvermögen und erhöhter mechanischer Festigkeit oder geringer Wärmeausdehnung führen.

Die nackte monolithische Struktur wird mit einer Katalysatorträgerschicht, die ein oder mehrere keramischen Oxide umfasst, oder einer Katalysatorschicht, die die katalytisch wirksamen Metalle und die fakultativen weiteren (Promotor-)Elemente bereits auf dem keramischen Oxidträgermaterial geträgert umfasst, beschichtet, wobei die Beschichtung nach einer Washcoat-Beschichtungsmethode hergestellt wird.

Im Folgenden werden bevorzugte Ausführungsvarianten für den Einbau der Monolithe in den Reaktor näher erläutert:

### Ausführungsvariante 1:

Die Monolithe werden ohne Beabstandung nebeneinander und übereinander, Monolith an Monolith, im Reaktor gestapelt, wobei die Durchströmung sämtlicher Monolithe in vertikaler Richtung gewährleistet sein muss.

Fertigungstechnisch bedingt weisen die Monolithe Unebenheiten und Verzug auf, so dass zwischen jeweils unmittelbar benachbarten Monolithen unterschiedlich breite Spalte beim Stapeln entstehen. Dies führt zu Bypässen des Reaktionsgasgemisches. Daher ist es erforderlich, dass die Monolithe auch auf ihren Außenwänden eine katalytische Beschichtung aufweisen.

Die Monolithe müssen an die zylindrische Innenwand des Reaktors durch Zuschnitt, entsprechend der Krümmung des Reaktors, angepasst werden. Der Zuschnitt erfolgt bevorzugt bereits vor der Lieferung der Monolithe zum Einbau in den Reaktor, da auf diese Weise anfallender Staub sowie Reststücke unmittelbar dem Edelmetall-Recycling zugeführt werden können.

Die Monolithe werden über eine oder mehrere Lagen, ohne Abstand, unmittelbar übereinander oder gegeneinander versetzt eingebaut. Bevorzugt werden 5 bis 30 Lagen, insbesondere 15 bis 20 Lagen übereinander eingebaut.

In einer bevorzugten Ausführungsvariante sind die einzelnen Lagen gegeneinander um je 45 ° verdreht angeordnet eingebaut, so dass keine durchgehenden Spalte in der Packung entstehen. Die Lagen werden bevorzugt der Gestalt übereinander gelegt, dass die Eckpunkte der Monolithe der nächstfolgenden Lage auf den Punkt aufzuliegen kommen, an dem in der darunter befindlichen Lage vier Monolithe aneinander angrenzen.

Für den Einbau einer horizontalen Lage wird bevorzugt in der nachfolgend beschriebenen Weise vorgegangen:
Zunächst werden die zurechtgeschnittenen Randstücke am Innenmantel des Reaktors verlegt, anschließend werden die einzelnen Monolithe von außen nach innen eingebaut. Die letzten vier Monolithe, die die Mitte der Lage ausfüllen, werden zusammen eingefügt und drücken die restlichen Monolithe der Lage noch einmal fest in die im Randbereich zur Reaktorinnenwand hin eingebrachte Blähmattenabdichtung.

Bei der obigen Ausführungsvariante ist der Einbau von Temperaturüberwachungselementen schwierig, weil dieselben fertigungsbedingt Dicken aufweisen, die größer als die Kanalweiten der Monolithe sind und somit eine Durchströmung derselben verhindern. Es besteht jedoch die Möglichkeit Temperaturüberwachungselemente zwischen in die Spalte zwischen den Monolithen einzeln einzubauen, dieselben nach außen, in die Blähmattenabdichtung zu ziehen und von dort aus über Stutzen nach außerhalb des Reaktors zu führen. Eine andere Einbauvariante für Temperaturüberwachungselemente ist das Durchbohren der Monolithe und Einsetzen einer Thermohülse, in die dann ein Multithermoelement eingebaut wird.

### Ausführungsvariante 1a:

Bei der nachfolgenden Ausführungsvariante ist es möglich, Thermoelemente zur Temperaturüberwachung in einfacher Weise einzubauen, ohne dass hierdurch Monolithkanäle verlegt werden. Hierzu werden die Monolithe, wie unter Ausführungsvariante 1 beschrieben, eingebaut, jedoch wird eine Beabstandung zwischen jeweils unmittelbar übereinander folgenden Lagen durch Einbringen von dünnen Blechabstandhaltern gewährleistet, die z. B. in Form eines Rostes oder auch als Einzelelemente eingebaut werden. Dadurch werden Abstände zwischen jeweils übereinander befindlichen Monolithlagen im Bereich von 10 bis 50 mm, bevorzugt im Bereich von 10 bis 20 mm, gewährleistet.

### Ausführungsvariante 2:

Die Ausführungsvariante 2 entspricht der Ausführungsvariante 1a, d.h. es sind mehrere horizontale Lagen von Monolithen vorgesehen, die übereinander, durch Abstandhalter beabstandet, eingebaut sind. Die Ausführungsvariante 2 unterscheidet sich von der Ausführungsvariante 1a dahingehend, dass die Monolithe jeder Lage jeweils gegeneinander an den vom Reaktionsgasgemisch nicht durchströmten Seiten in Blähmatten oder Mineralfaservliesmatten eingefüllt und somit gegeneinander abgedichtet sind.

### Ausführungsvariante 3:

In der Ausführungsvariante 3 wird von Modulen aus zwei oder mehreren, nebeneinander und/oder übereinander gestapelten Monolithen ausgegangen, wobei die Monolithmodule so zu dimensionieren sind, dass sie noch über die Mannlöcher (Zugangsstutzen) in den Reaktor einbaubar sind. Die einzelnen Module sind an ihrem Umfang, unter Freilassung der Durchtrittsöffnungen für das Reaktionsgasgemisch durch die Monolithkanäle, in einer Blähmatte oder in einem Mineralfaservlies, insbesondere aus einer Keramikfaser bevorzugt aus polykristallinen Mullitfasern eingehüllt und in eine metallische Einhausung mit Verspanneinrichtung eingesetzt. Die einzelnen Module werden nebeneinander und unter Einbau von Blechabstandhaltern, wie unter Ausführungsvariante 1 a beschrieben, in Lage übereinander angeordnet.

Der Reaktor wird bei Temperaturen zwischen 500 und 690 °C, bevorzugt 550 bis 620 °C betrieben und von unten nach oben durchströmt. Während des Reaktorbetriebes dehnt sich die metallische Reaktorhülle stärker aus, als die keramischen Monolithe, wodurch diese lose werden können.

Erfindungsgemäß ist daher die Innenwand des Reaktors im Bereich der Monolithe mit einer druckstabilen Isolierung ausgekleidet und die Monolithe werden gegen dieselbe mittels Blähmatten abgedichtet.

Ein Vorteil der Innenwandisolierung ist die Verringerung der Reaktorwandtemperatur und somit eine geringere thermische Ausdehnung derselben. Des Weiteren lässt sich wegen der geringeren Reaktorwandtemperatur ein preiswerterer Werkstoff für den Reaktormantel wählen und die Blähmatten müssen im Randbereich weniger Abdichtungsleistung erbringen.

Die Monolithe nach der Ausführungsvariante 1 und 1a müssen auf ihrer gesamten Oberfläche, d.h. sowohl in den Kanälen als auch auf ihrer Außenfläche, beschichtet sein.

Die makroporöse Struktur von Keramikmonolithen erleichtert die Verankerung der Washcoatschicht. Die Art und Weise der Washcoat-Beschichtung kann in zwei Methoden unterteilt werden: Man kann den makroporösen Träger (teilweise) mit dem eine große Oberfläche aufweisenden Washcoatmaterial füllen oder einen Washcoat als Schicht in den Poren des Keramikträgers abscheiden. Das Porenfüllen führt zur stärksten Wechselwirkung zwischen Monolith und Washcoat, da der größte Teil der Washcoatschicht tatsächlich in den Poren des Trägers fixiert ist und nicht nur an die äußere Oberfläche der Monolithkanäle gebunden ist. Diese Art von Beschichtung wird mit einer Lösung (oder einem Sol) des abzuscheidenden Materials oder mit einer sehr kleine kolloidale Teilchen enthaltenden Lösung durchgeführt. Der Nachteil des Beschichtens mittels Porenfüllung besteht darin, dass die abscheidbare Beschichtungsmenge begrenzt ist, da die Poren irgendwann vollständig gefüllt sein werden und der Washcoat unzugänglich werden wird.

Monolithe bieten günstige Voraussetzungen für die Durchführung der autothermen Dehydrierung von Kohlenwasserstoffen: insbesondere sind engere Reaktorquerschnitte und höhere Strömungsgeschwindigkeiten gegenüber regellos gepackten Festbetten realisierbar, so dass eine effektive, gestufte Zudosierung des Sauerstoffes in den Kohlenwasserstoff enthaltenden Hauptstrom möglich ist. Aufgrund des dadurch, gegenüber regellos gepackten Festbetten, kleineren Reaktorquerschnittes sind sowohl die Verteiler als auch die festen Einbauten der Mischzonen mechanisch weniger stark belastet, d.h. sie hängen wegen der geringeren Verankerungslänge weniger stark durch. Darüber hinaus ist die Hauptströmungsrichtung durch den Reaktor nicht auf eine Abwärtsströmung begrenzt, wie im Fall von regellos gepackten Festbetten.

Nach längerer Standzeit können die in der vorliegenden Schrift empfohlenen Katalysatoren normalerweise auf einfache Art und Weise regeneriert werden, beispielsweise indem man zunächst in ersten Regenerationsstufen Luft, die (vorzugsweise) mit Stickstoff und/oder Wasserdampf verdünnt ist, bei einer Eintrittstemperatur von 300 bis 600 °C (in Extremfällen auch bis zu 750 °C), häufig von 500 bis 600 °C, durch das Katalysatorfestbett leitet. Die Katalysatorbelastung mit Regenerationsgas kann (bezogen auf die Gesamtmenge an regeneriertem Katalysator) beispielsweise 50 bis 10 000 h⁻¹ betragen, und der Sauerstoffgehalt des Regenerationsgases kann 0,5 bis 20 Vol.-% betragen.

Danach ist es im Allgemeinen empfehlenswert, auch unter ansonsten identischen Bedingungen mit reinem molekularem Wasserstoff oder mit molekularem Wasserstoff, der mit Inertgas (vorzugsweise Wasserdampf und/oder Stickstoff) verdünnt ist, zu regenerieren (der Wasserstoffgehalt sollte ≥ 1 Vol.-% sein).

Die nebeneinander und übereinander ohne Abstand zu einer Packung gestapelten Monolithe sind bevorzugt in einer Blähmatte oder in einem Mineralfaservlies eingehüllt und in eine Einhausung mit Verspanneinrichtung eingesetzt. Als Mineralfaservliese werden bevorzugt Vliese eingesetzt, wie sie für den Einsatz für Abgaskatalysatoren bekannt sind, beispielsweise Interam^{®} Lagermatten der Firma 3M^{®}.

Blähmatten sind aus der katalytischen Abgasreinigung bekannt und beispielsweise in DE-A 40 26 566 beschrieben: Sie bestehen im Wesentlichen aus Keramikfasern mit Glimmereinlagerung, insbesondere Vermiculit. Infolge der Glimmereinlagerung hat die Blähmatte bei steigenden Temperaturen das Bestreben sich auszudehnen, wodurch eine besonders sichere Halterung des darin eingehüllten Körpers auch bei höheren Temperaturen erreicht wird.

Die Mineralvliese oder Blähmatten werden so ausgewählt, dass sie sich unter Wärmeeinwirkung ausdehnen und dass sie die in der Regel keramischen Monolithe gegen das Gehäuse abdichten, insbesondere eine Reibung der Monolithe am Gehäuse sowie eine Bypassströmung des Reaktionsgasgemisches an der Innenwand des Gehäuses verhindern.

Die Blähmatten im Randbereich, in die die Monolithe eingehüllt sind, sorgen für eine stabile Position derselben, da sie unter Wärmeausdehnung eine Spannkraft erzeugen. Im Zuge von Fehlbedienungen kann die Spannkraft jedoch nachlassen. Daher kann vorteilhaft eine Verspanneinrichtung vorgesehen werden.

Die Packungen aus neben- und/oder übereinander gestapelten Monolithen können vorteilhaft aus vier oder mehreren Teilpackungen gebildet sein, die jeweils einzeln mit einer metallischen Einfassung versehen sind und zusammensetzbar sind, dergestalt, dass sie den Querschnitt des Reaktors vollständig ausfüllen und gegeneinander gegen Bypässe abdichtbar sind.

Alternativ oder zusätzlich ist es möglich, Packungen aus zwei oder mehreren Teilpackungen über die Höhe derselben zusammenzusetzen.

Vorteilhaft können sämtliche obigen Teilpackungen in einem Gehäuse zusammengefasst sein, um die Handhabbarkeit zu erleichtern.

Die Packungen aus neben- und/oder übereinander gestapelten Monolithen liegen insbesondere jeweils auf einem Auflagerost auf.

Die Auflageroste sind vorteilhaft so auszugestalten, dass sie die Kanäle für die Durchströmung durch das Reaktionsgasgemisch nicht versperren. Um dies zuverlässig zu verhindern ist es vorteilhaft, im unmittelbar an den Auflagerost angrenzenden Bereich eine oder mehrere Lagen von Monolithen vorzusehen, die einen wesentlich größerem Querschnitt der Kanäle gegenüber den übrigen, vom Auflagerost weiter entfernt liegenden Monolithen, aufweisen. Die Stegdicke zwischen den Kanälen muss so dünn sein, dass die Stege die darüberliegenden Kanäle nicht verstopfen.

Alternativ können auf den Auflagerost eine oder mehrere Lagen eines Drahtnetzes vorgesehen sein, wobei die Maschen der unmittelbar auf dem Auflagerost befindlichen Lage etwas gröber sind und zu den Monolithen hin zunehmend feiner werden. Bevorzugt sind Maschenweiten von 5 bis 15 mm und Drahtdurchmesser von 0,2 bis 2 mm.

Zusätzlich oder alternativ kann im unmittelbar an den Auflagerost angrenzenden Bereich eine Lage aus einer offenporigen Schaumkeramik bevorzugt mit einem durchströmbaren Lückenvolumen von 70 bis 90 % vorgesehen sein.

Besonders bevorzugt kann im unmittelbar an den Auflagerost angrenzenden Bereich eine erste Lage aus einer hochporösen Schaumkeramik, insbesondere mit einem freien Lückenvolumen von ca. 70 % und einer Höhe im Bereich von 10 bis 100 mm, bevorzugt im Bereich von 40 bis 60 mm, vorgesehen sein, und darüber eine zweite Lage, die aus Monolithen mit einer Dicke von 50 mm gebildet ist, die Kanäle mit größerem Querschnitt gegenüber den übrigen, vom Auflagerost weiter entfernt liegenden Monolithen, aufweisen.

Die Innenwand des Reaktors ist durchgehend, d.h. über dessen gesamte Länge, komplett, mit einer Isolationsschicht versehen.

Im Bereich der katalytisch aktiven Zonen muss die Isolationsschicht der Innenwand druckstabil und möglichst gasdicht sein. Bevorzugt ist die Isolationsschicht im Bereich der katalytisch aktiven Zonen doppellagig ausgeführt, mit einer ersten an der Innenwand des Reaktors anliegenden, druckstabilen Lage, sowie einer zweiten, zum Reaktorinnenraum hin ausgerichteten Lage, die aus einer Blähmatte gebildet ist.

Bevorzugt ist zwischen der an der Innenwand des Reaktors vorgesehenen, druckstabilen Lage eine weitere, gegenüber derselben, deutlich dünnere Lage, die aus einer Blähmatte gebildet ist, vorgesehen, um zu gewährleisten, dass die druckstabile Lage möglichst gut an der Innenwand des Reaktors anliegt.

In den übrigen Bereichen der Reaktorinnenwand, d.h. im Bereich der Verteiler und Mischzonen ist die Isolationsschicht bevorzugt einlagig, aus einer hochtemperaturstabilen Fasermatte, insbesondere aus einer polykristallinen Mullitfaser, ausgebildet, die zum Reaktorinnenraum hin eine Blechverkleidung aufweist, um zu verhindern, dass das Reaktionsgemisch in die Isolationsschicht eindringt.

Der zu dehydrierende kohlenwasserstoffhaltige Gasstrom wird am unteren Ende des Reaktors in denselben eingeleitet und strömt von unten nach oben durch den Reaktor.

Vorteilhaft wird der zu dehydrierende kohlenwasserstoffhaltige Gasstrom vorgeheizt, vorteilhaft in einem Wärmetauscher, der oberhalb der obersten katalytisch aktiven Schicht im Reaktor oder außerhalb desselben angeordnet ist, wobei der zu dehydrierende kohlenwasserstoffhaltige Gasstrom über eine Zuführleitung in den Wärmetauscher eingeleitet, im Wärmetauscher durch das Reaktionsgasgemisch im Gegenstrom durch indirekten Wärmetausch aufgeheizt und weiter an das dem Wärmetauscher entgegengesetzte untere Ende des Reaktors geleitet, dort umgelenkt, über einen Stutzen in den Reaktor eingeleitet, und in den Mischzonen mit dem Sauerstoff enthaltenden Gasstrom vermischt wird, worauf im Reaktor die autotherme Gasphasendehydrierung in den katalytisch aktiven Zonen stattfindet.

Der in den Reaktor integrierte Wärmetauscher kann insbesondere als Rohrbündel- oder Plattenwärmetauscher in Gegenstromfahrweise ausgebildet sein.

Der Rohrbündelwärmetauscher ist vorteilhaft aus einem hoch warmfesten Edelstahl, insbesondere einem Edelstahl mit der Werkstoffnummer 1.4541 oder 1.4910, gebildet. Die Rohre des Rohrbündelwärmetauschers sind an beiden Enden derselben in Rohrböden, vorteilhaft spaltfrei, durch Hinterbodenschweißung eingebracht und die Rohrböden des Rohrbündelwärmetauschers auf der Heißgasseite derselben mit einem hitzebeständigen Edelstahl, insbesondere mit einem Edelstahl mit der Werkstoffnummer 1.4841, plattiert. Besonders vorteilhaft ist ein Wärmetauscher in Schwimmkopfausführung.

Vorteilhaft kann der zu dehydrierende kohlenwasserstoffhaltige Gasstrom an zwei oder mehreren Stellen in den Wärmetauscher eingeleitet wird, bevorzugt als ein Hauptstrom mit höherem Massenstrom und ein oder mehrere Nebenströme mit niedrigerem Massenstrom gegenüber dem Hauptstrom.

Für die Inbetriebnahme des Reaktors ist es vorteilhaft, den Wärmetauscher bypassen zu können:
Im Folgenden wird die Inbetriebnahme des Reaktorsystems, d.h. das Aufheizen desselben auf die Reaktionstemperatur der autothermen Gaswasserdehydrierung beschrieben.

Das Reaktorsystem, d.h. der Reaktor, der Wärmetauscher und die Verbindungsleitungen, hat zunächst Umgebungstemperatur, und müssen auf die Betriebstemperatur der autothermen Gaswasserdehydrierung, im Falle der Butandehydrierung, auf ca. 550 °C gebracht werden.

Schritt 1: Aufheizung des Reaktors auf ca. 200 °C.

Das Reaktorsystem wird mittels eines Aufheizgases, das beispielsweise Kreisgas oder Stickstoff sein kann und das über eine Feedleitung, mit ca. 230 °C, zugeführt wird, aufgeheizt. Dabei wird der Wärmetauscher mantelseitig kurzgeschlossen (gebypassed), d.h. das Aufheizgas strömt direkt in den Reaktor, heizt denselben auf ca. 200 °C auf, und strömt anschließend lediglich durch die Rohre des Wärmetauschers, die es ebenfalls aufheizt und verlässt anschließend das Reaktorsystem. Der die Rohre des Wärmetauschers umgebende Mantelraum wird vom Heizgas nicht durchströmt.

Schritt 2: Spülen des Mantelraumes, damit derselbe weitgehend sauerstofffrei wird.

Am rohrseitigen Austritt aus dem Wärmetauscher ist eine Temperaturmesseinrichtung vorgesehen. Sobald dieselbe eine Temperatur von ca. 200 °C anzeigt, wird das Bypassing des Wärmetauschers aufgehoben und das Aufheizgas mantelseitig im Gegenstrom durch den Wärmetauscher geführt. Hierbei wird der Mantelraum gespült. Schritt 3: Weitere Aufheizung des Reaktorsystems mittels eines Brenngases, das im Reaktor verbrannt wird.

In der Reaktorzuleitung sind eine oder mehrere Brenngaszuführungen mit entsprechenden nachgeschalteten Mischorganen vorgesehen. Als Brenngas eignet sich insbesondere Wasserstoff, Erdgas oder auch der zu dehydrierende Kohlenwasserstoff. Besonders bevorzugt ist Wasserstoff, der an der edelmetallhaltigen Katalysatorbeschichtung bereits ab ca. 200 °C zündet, und den Reaktor auf die erforderliche Betriebstemperatur von insbesondere ca. 550 °C aufheizt. Hierfür kann ein sauerstoffhaltiger Strom, Magerluft oder besonders bevorzugt Luft als Feedgas zugefahren werden. Der Abstand der Eindüsung des Brenngases zum edelmetallhaltigen Katalysator soll so kurz wie möglich sein. Die Konzentration des eingesetzten Brenngases ist insbesondere dergestalt zu begrenzen, dass die Gaszusammensetzung im Reaktorsystem außerhalb des Explosionsbereiches und insbesondere außerhalb des Detonationsbereiches unter den vorliegenden Betriebsbedingungen liegt. Die Mindestbrenngaskonzentration ist bevorzugt ebenfalls vorzugeben, und zwar der Gestalt, dass die durch Verbrennung am Eintritt in den Reaktor erreichte Temperaturerhöhung ausreicht, um die Temperatur des kalten Feedgases im rekuperativen Wärmetauscher zu erhöhen. Für den Fall, dass Wasserstoff als Brenngas eingesetzt wird, ist eine Konzentration von ca. 1,4 Vol.-% Wasserstoff besonders bevorzugt.

Bevorzugt sind zusätzlich zum Wärmetauscher eine oder mehrere Zusatzheizungen für den zu dehydrierenden kohlenwasserstoffhaltigen Gasstrom vorgesehen.

Besonders bevorzugt ist als Zusatzheizung eine elektrische Heizung vorgesehen, die insbesondere lösbar, als Einstecksystem oder als Muffelbrenner, in den zu dehydrierenden kohlenwasserstoffhaltigen Gasstrom nach dem Austritt desselben aus dem Wärmetauscher.

Der kohlenwasserstoffhaltige Gasstrom strömt von unten über einen Stutzen in den Reaktor ein und wird vor jeder katalytisch aktiven Zone jeweils in einer Mischzone mit festen Einbauten gleichgerichtet, so dass nach der Mischzone die Strömungsgeschwindigkeit desselben über den Reaktorquerschnitt eine Abweichung von maximal +/- 2 % vom Mittelwert aufweist.

Der Sauerstoff enthaltende Gasstrom wird über eine oder mehrere, unabhängig voneinander regelbare Zuführleitungen, wobei jede Zuführleitung eine oder mehrere Verteiler versorgt, in jede der Mischzonen zugeführt.

Die Verteiler können insbesondere als Ringverteiler oder Parallelstabverteiler ausgebildet sein.

Da die Verweilzeit nach der Einbringung des Sauerstoff enthaltenden Gases zum kohlenwasserstoffhaltigen Gasstrom sehr gering, insbesondere unter 60 ms, sein soll, sind zusätzliche Einrichtungen wie Mischplatten, Metallstreifen in langgestreckter oder Ringform vorteilhaft, um die Intensität der Vermischung zu erhöhen.

Die Zuführleitungen für den Sauerstoff enthaltenden Gasstrom sind vorteilhaft thermisch kompensiert und mit Haltelaschen an der Reaktorwand befestigt.

Bevorzugt umfasst jede Mischzone jeweils einen Rohrverteiler, gebildet aus einer Vielzahl von parallel zueinander, in einer Ebene senkrecht zur Längsrichtung des Reaktors angeordneten Einsteckrohren, die mit einer oder mehreren Verteilerkammern verbunden sind, und die eine Vielzahl von gleichmäßig zueinander beabstandeten Austrittsöffnungen für den Sauerstoff enthaltenden Gasstrom aus den Einsteckrohren aufweisen, sowie einer Vielzahl von gleichmäßig zueinander beabstandeten Mischkörpern.

Besonders bevorzugt sind die Mischkörper als Mischplatten ausgebildet.

Erfindungsgemäß sind Mannlöcher, d.h. Zugangsstutzen, die dergestalt dimensioniert sind, dass ein Arbeiter durch dieselben in den Reaktorinnenraum gelangen kann, vorgesehen, über die die eine oder jede der mehreren katalytisch aktiven Zonen von außerhalb des Reaktors zugänglich ist. Dadurch ist, in Verbindung mit der aufrechtstehenden Anordnung des Reaktors, jede katalytisch aktive Zone einzeln von außerhalb des Apparates zugänglich, ohne das hierfür Teile desselben herausgenommen werden müssten. Durch den einfachen Zugang über Mannlöcher ist es möglich, jede der katalytisch aktiven Packungen sowie die entsprechenden Auflageroste einzeln aus- bzw. einzubauen.

Einen besonders zeitsparenden, einfachen Katalysatoraustausch gewährleistet eine Ausführungsform des Reaktors zur Durchführung einer autothermen Gasphasendehydrierung eines kohlenwasserstoffhaltigen Gasstromes mit einem Sauerstoff enthaltenden Gasstrom unter Erhalt eines Reaktionsgasgemisches, an einem heterogenen Katalysator, der als Monolith ausgebildet ist, wobei der Reaktor die , Form eines Zylinders mit vertikaler Längsachse aufweist und wobei
- im Innenraum des Reaktors eine oder mehrere katalytisch aktive Zonen angeordnet sind, umfassend jeweils eine Packung aus neben- und/oder übereinander gestapelten Monolithen und wobei vor jeder katalytisch aktiven Zone jeweils eine Mischzone mit festen Einbauten vorgesehen ist,
- mit einer oder mehreren Zuführleitungen für den zu dehydrierenden kohlenwasserstoffhaltigen Gasstrom am unteren Ende des Reaktors,
- mit einer oder mehreren, unabhängig voneinander regelbaren Zuführleitungen, wobei jede Zuführleitung einen oder mehrere Verteiler versorgt, für den Sauerstoff enthaltenden Gasstrom in jede der Mischzonen sowie
- mit einer oder mehreren Abführleitungen für das Reaktionsgasgemisch der autothermen Gasphasendehydrierung am oberen Ende des Reaktors
wobei die Innenwand des Reaktors durchgehend mit einer Isolationsschicht versehen ist,
und wobei die Zugänglichkeit der einen oder jeder der mehreren katalytisch aktiven Zonen gewährleistet ist, indem hierfür keine Mannlöcher vorgesehen sind, jedoch die eine oder jede der mehreren katalytisch aktiven Zonen, umfassend jeweils eine Packung aus neben- und/oder übereinander gestapelten Monolithen einschließlich der vor jeder katalytisch aktiven Zone vorgesehenen Mischzone mit festen Einbauten, der einen oder mehreren, unabhängig voneinander regelbaren Zuführleitungen, sowie einschließlich der einen oder mehreren Verteiler, jeweils ein Bauelement bilden, das einzeln montier- und demontierbar ist.

In dieser Ausführungsform sind die einzelnen Bauelemente jeweils einzeln, weitgehend komplett, insbesondere durch Lösen entsprechender Flansche, ausbaubar, beispielweise sobald eine Deaktivierung des Katalysators dies erforderlich macht, wobei zusätzlich lediglich die erforderlichen Anschlussleitungen sowie Mess- und Regeleinrichtungen gelöst werden müssen. Entsprechend einfach ist auch die erneute Montage des einzelnen Bauelementes nach Austausch der den Katalysator bildenden oder diesen beinhaltenden Monolithe.

In dieser Ausgestaltung kann die Rüstzeit gegenüber einem gleichartigen Reaktor, der jedoch über Mannlöcher, bestückt wird, bis auf 1/10 reduziert werden.

Darüber hinaus lässt sich durch Wegfall der Mannlöcher der katalysatorfreie Zwischenraum zwischen aufeinanderfolgenden Monolithpackungen deutlich reduzieren, so dass ein Reaktor bei gleicher Kapazität und geringerer Bauhöhe gebaut werden kann und dass darüber hinaus die Verweilzeit des Reaktionsgemisches in den katalysatorfreien Zwischenräumen auf Grund des reduzierten Volumens derselben niedriger wird, was insbesondere von Vorteil ist, da unkontrollierbare Nebenreaktionen nicht oder in deutlich geringerem Ausmaß stattfinden.

Gegenstand der Erfindung ist auch ein Verfahren zur Durchführung einer autothermen Gasphasendehydrierung unter Verwendung des obigen Reaktors.

Bevorzugt werden zwei oder mehrere Reaktoren eingesetzt, wobei mindestens ein Reaktor für die autotherme Gasphasendehydrierung genutzt und gleichzeitig mindestens ein weiterer Reaktor regeneriert wird.

Bevorzugt ist die autotherme Gasphasendehydrierung eine Dehydrierung von Propan, von Butan, von Isobutan, von Buten zu Butadien, von Ethylbenzol zu Styrol oder von Ethan zu Ethylen.

Der erfindungsgemäße Reaktor ist in technisch einfacher Weise realisierbar; da die Reaktorwand zylindrisch ist, ist es möglich, an beiden Enden derselben Kugelböden einzusetzen, die einfach und preiswert sind. Dadurch lässt sich der Reaktor in wirtschaftlicher Weise druckstoßfest bauen.

Dadurch, dass der Reaktormantel an der Innenwand eine durchgehende Isolationsschicht aufweist, ist es möglich, denselben aus weniger anspruchsvollen und somit preiswerteren Werkstoffen herzustellen.

Die Erfindung wird im Folgenden anhand einer Zeichnung erläutert.

In der Zeichnung zeigen im Einzelnen:
Figur 1 eine schematische Darstellung einer bevorzugten Ausführungsform eines erfindungsgemäßen Reaktors,
Figur 2 eine weitere bevorzugte Ausführungsform eines erfindungsgemäßen Reaktors mit integriertem Wärmetauscher, mit einer Detaildarstellung in Figur 2A
Figur 3 eine weitere bevorzugte Ausführungsform eines erfindungsgemäßen Reaktors mit einer Einrichtung zum Aufheizen mit mantelseitigem Bypass des Wärmetauschers,
Figur 4 eine weitere bevorzugte Ausführungsform eines erfindungsgemäßen Reaktors mit außerhalb des Reaktors vertikal angeordnetem Wärmetauscher,
Figur 5 eine weitere bevorzugte Ausführungsform eines erfindungsgemäßen Reaktors mit außerhalb desselben, horizontal angeordnetem Wärmetauscher,
Figur 6 einen Ausschritt aus einer bevorzugten Ausführungsform eines erfindungsgemäßen Reaktors,
Figur 7 eine schematische Darstellung einer Ausführungsform des erfindungsgemäßen Reaktors mit einzeln, durch Flanschen verbundenen Bauelementen, der von unten nach oben durch das Reaktionsgemisch durchströmt ist, mit Detaildarstellungen in Figur 7A und Figur 7B,
Figur 8 eine schematische Darstellung einer Ausführungsform eines erfindungsgemäßen Reaktors analog Figur 7, jedoch mit Durchströmung des Reaktionsgemisches von oben nach unten und
Figur 9 eine schematische Darstellung einer Ausführungsform eines erfindungsgemäßen Reaktors analog Figur 7, außerhalb des Reaktors vertikal angeordneten Wärmetauscher.

In den Figuren bezeichnen gleiche Bezugszeichen jeweils gleiche oder entsprechende Merkmale.

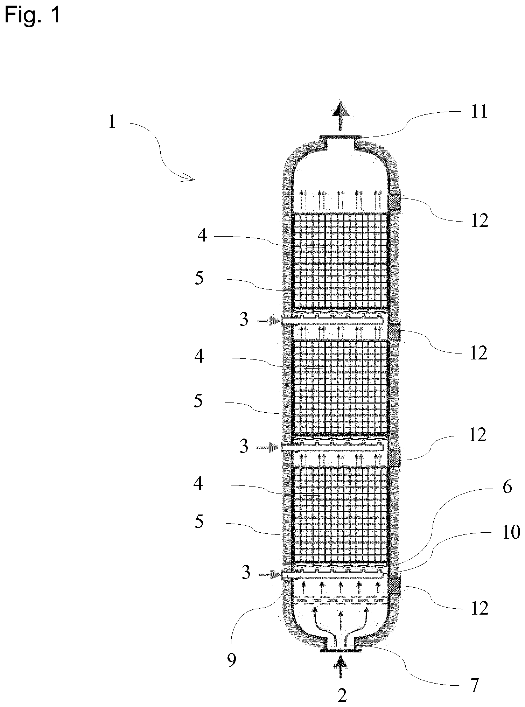

Die schematische Darstellung in Figur 1 zeigt einen Reaktor 1 mit Zuführung eines kohlenwasserstoffhaltigen Gasstromes 2 über eine Zuführleitung 7 am unteren Ende desselben. Sauerstoff enthaltende Gasströme 3 werden über Zuführleitungen 9 in jede der Mischzonen 6 eingeleitet, an die sich jeweils eine katalytisch aktive Zone 5 aus neben- und übereinander gestapelten Monolithen 4, anschließt. Das Reaktionsgasgemisch verlässt den Reaktor über den Stutzen 11 am oberen Ende desselben.

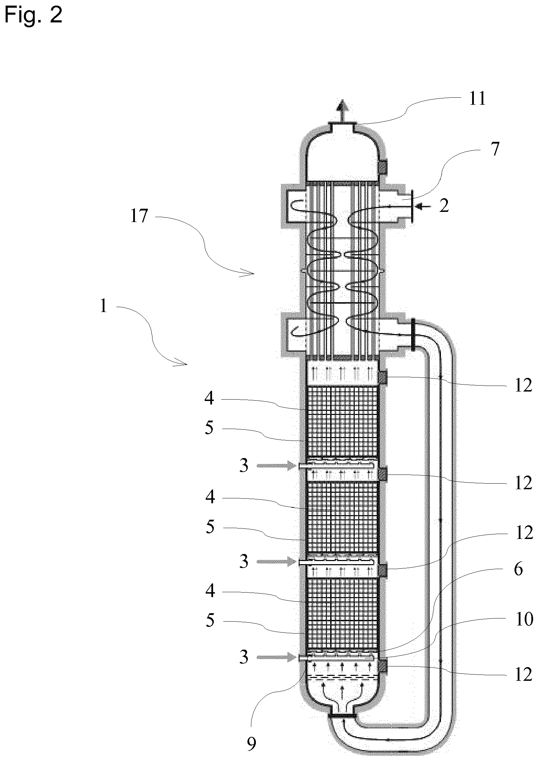

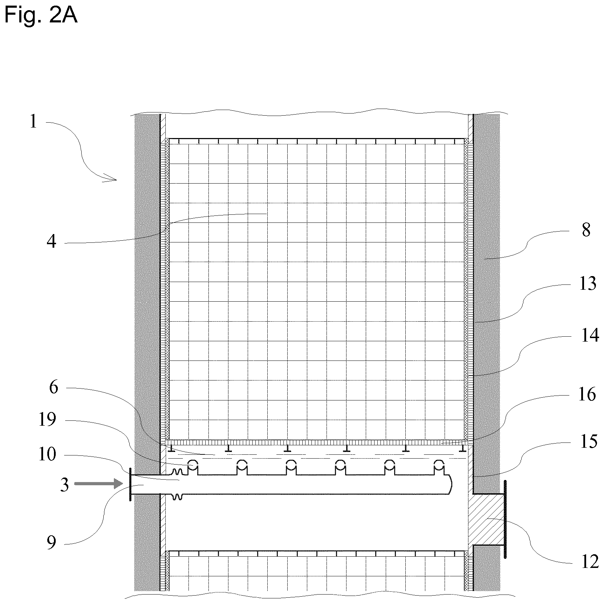

Die in Figur 2 dargestellte Ausführungsform unterscheidet sich von der Ausführungsform in Figur 1 insofern, als ein in den Reaktor 1 am oberen Ende desselben integrierter Wärmetauscher vorgesehen ist.

Die Detaildarstellung in Figur 2A verdeutlicht die einzelnen technischen Elemente einer katalytisch aktiven Zone 5, einschließlich der Zuführleitung 9 für den Sauerstoff enthaltenden Gasstrom 3 sowie des Mannloches 12, das die Zugänglichkeit zu den Monolithen der Packung in der katalytisch aktiven Zone 5 gewährleistet.

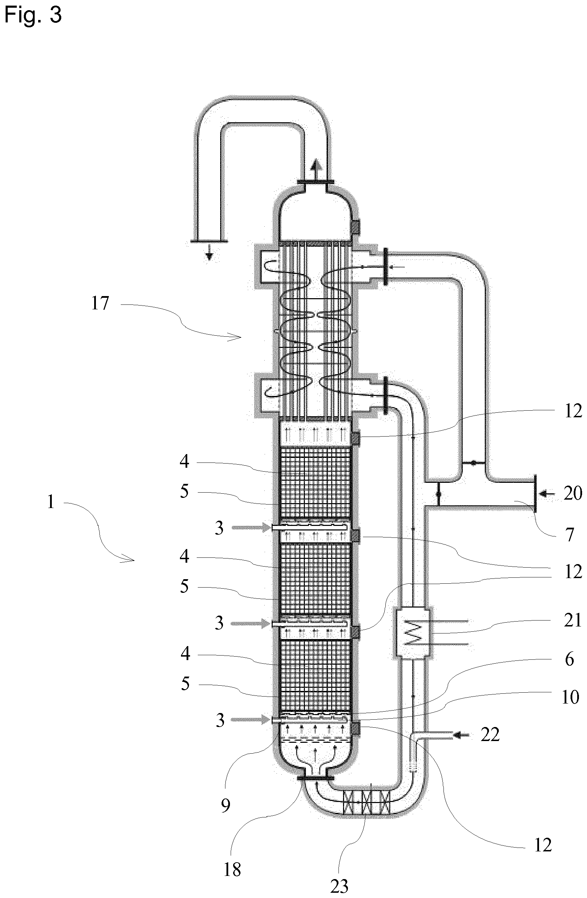

Die in Figur 3 dargestellte Ausführungsform umfasst zusätzlich Einrichtungen für das Aufheizen des Reaktorsystems auf Betriebstemperatur, mit mantelseitigem Bypass des Wärmetauschers 17.

Das Heizgas 20 strömt zunächst von unten nach oben durch den Reaktor 1 und anschließend durch die Rohre des Wärmetauschers 17. Sobald der am oberen Ende des Reaktors aus demselben austretende Gasstrom eine Temperatur von ca. 200 °C erreicht hat, wird der mantelseitige Bypass des Wärmetauschers 17 aufgehoben, und das Heizgas strömt auch durch den Mantelraum des Wärmetauschers. In der Zuleitung des Heizgases zum Reaktor 1 ist ein zusätzlicher Wärmetauscher 21 vorgesehen. Für die weitere Aufheizung des Reaktorsystems wird ein Brenngas 22 über einen Mischer 23 in den Reaktor 1, am unteren Ende desselben, eingeleitet.

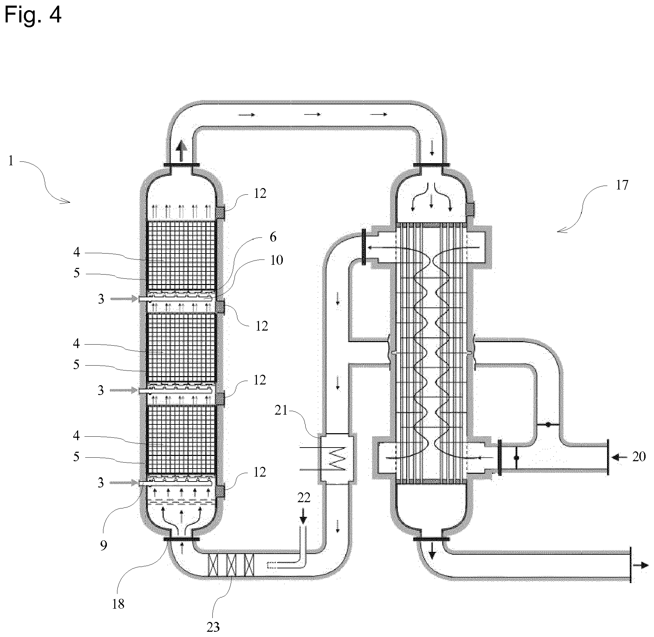

Figur 4 zeigt eine weitere bevorzugte Ausführungsform eines erfindungsgemäßen Reaktors 1, mit seitlich, außerhalb des Reaktors 1, vertikal angeordnetem Wärmetauscher 17.

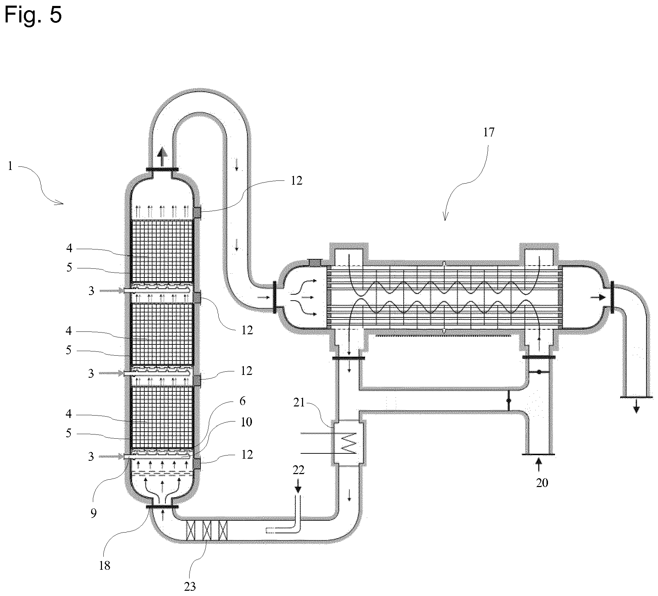

Figur 5 zeigt eine weitere bevorzugte Ausführungsform eines erfindungsgemäßen Reaktors, mit seitlich, außerhalb des Reaktors 1, horizontal angeordnetem Wärmetauscher 17.

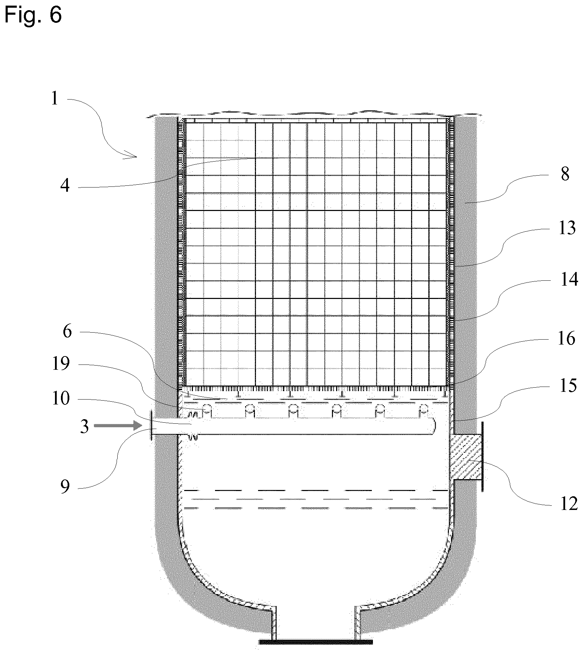

Der in Figur 6 dargestellte Ausschnitt verdeutlicht den Aufbau der an der Reaktorinnenwand vorgesehenen Isolationsschicht, die im Bereich der katalytisch aktiven Zonen 5 zweilagig ausgebildet ist, mit einer der Reaktorinnenwand anliegenden ersten Lage 13 aus einem druckstabilen Material, sowie einer zweiten zum Reaktorinnenraum hin ausgerichteten Lage aus einer Blähmatte.

In den übrigen Bereichen ist eine einlagige Isolationsschicht 15 vorgesehen, die aus einer Fasermatte gebildet ist, und die zum Reaktorinnenraum hin eine Blechverkleidung aufweist.

Der Ausschnitt verdeutlicht die Zuführung des sauerstoffenthaltenden Gasstromes 3 über einen Verteiler 10, gebildet aus einer Vielzahl von in Längsrichtung des Reaktors angeordneten Einsteckrohren 19.

Die Figur zeigt ferner den Auflagerost 16.

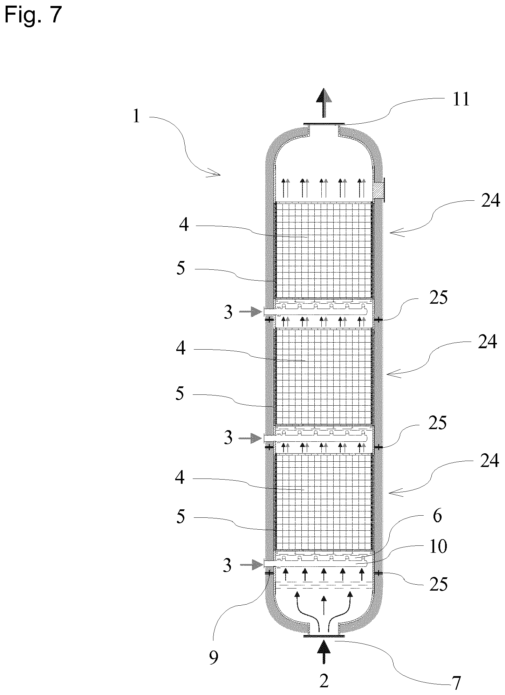

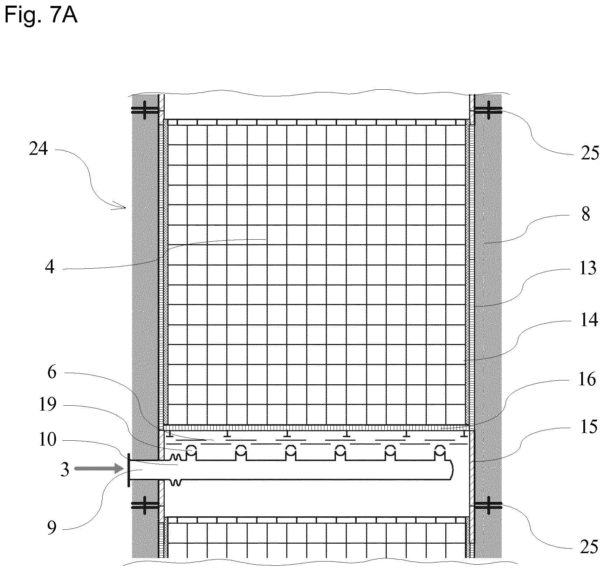

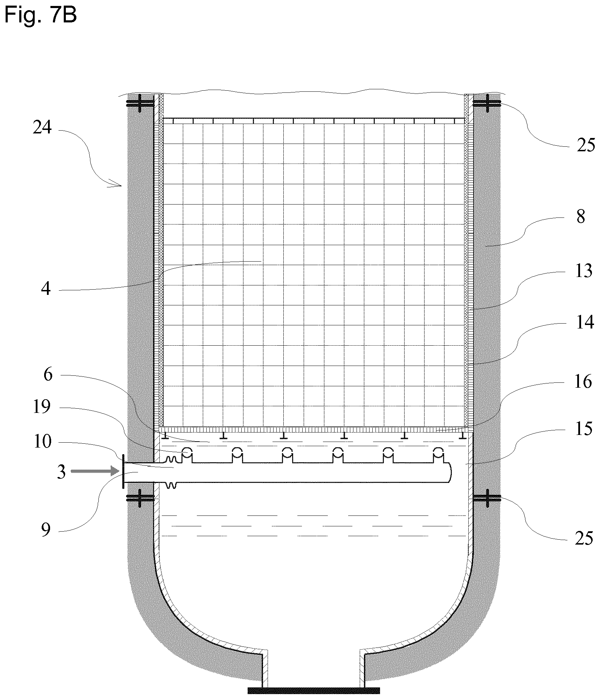

Figur 7 zeigt eine Ausführungsform eines erfindungsgemäßen Reaktors in einer Bauweise mit beispielhaft drei einzeln montier- und demontierbaren Bauelementen 24 mit Detaildarstellung eines mittleren Bauelementes 24 in Figur 7A sowie eines unteren Bauelementes 24 in Figur 7B.

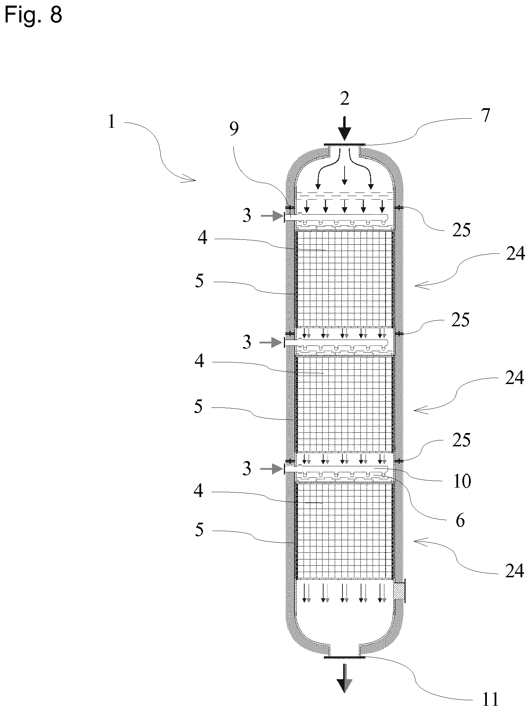

Der in Figur 8 dargestellte Reaktor 1 ist analog Reaktor 1 in Figur 7, jedoch mit umgekehrter Strömungsführung für das Reaktionsgemisch, und entsprechend auch geänderter Anordnung der Zuführleitungen 9 für den Sauerstoff enthaltenden Gasstrom 3.

Beide in Figur 7 sowie Figur 8 dargestellten Reaktoren 1 sind aus einzeln montier- und demontierbaren Bauelementen 24 zusammengebaut.

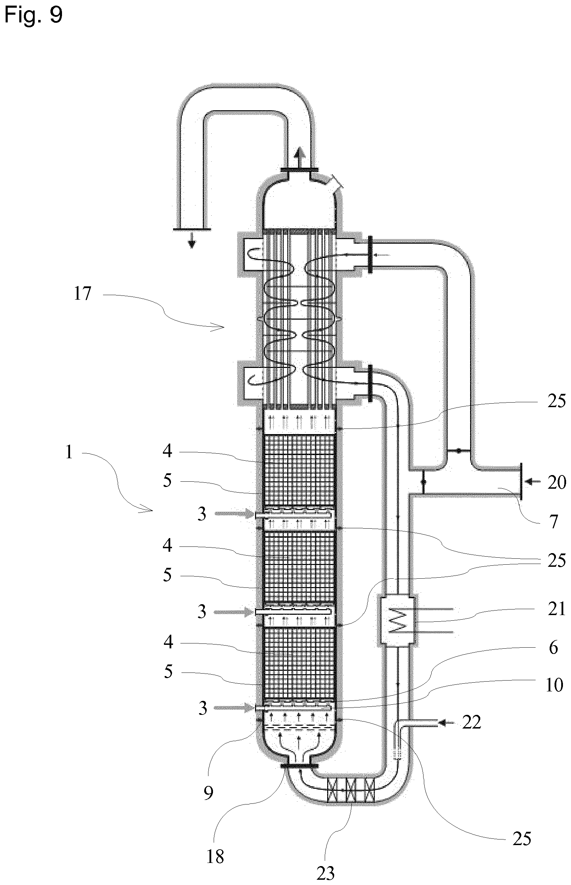

Der in Figur 9 dargestellte Reaktor 1 entspricht dem in Figur 3 dargestellten Reaktor 1, wobei jedoch für den Zugang zu den Monolithen keine Mannlöcher vorgesehen sind, sondern die einzelnen Bauelemente 24 lösbar, über Flansche 25 miteinander verbunden sind.

### Bezugszeichenliste

- 1: Reaktor
- 2: Gasstrom, Kohlenwasserstoffe enthaltend
- 3: Gasstrom, Sauerstoff enthaltend
- 4: Monolith(e)
- 5: katalytisch aktive Zone
- 6: Mischzone
- 7: Zuführleitung für Gasstrom 2
- 8: äußere Isolierung
- 9: Zuführleitung für Gasstrom 3
- 10: Verteiler
- 11: Abführleitung
- 12: Mannloch
- 13: formstabile Isolierung
- 14: Abdichtung
- 15: Schutzisolierung
- 16: Auflagerost
- 17: Wärmetauscher
- 18: Stutzen
- 19: Einsteckrohre
- 20: Heizgas
- 21: zusätzlicher Wärmetauscher
- 22: Brenngas
- 23: Mischer
- 24: Bauelement
- 25: Flansch

## Patentansprüche

1. Reaktor (1) in Form eines Zylinders mit vertikaler Längsachse zur Durchführung einer autothermen Gasphasendehydrierung eines kohlenwasserstoffhaltigen Gasstromes (2) mit einem Sauerstoff enthaltenden Gasstrom (3) unter Erhalt eines Reaktionsgasgemisches, an einem heterogenen Katalysator, der als Monolith (4) ausgebildet ist, wobei
- im Innenraum des Reaktors (1) eine oder mehrere katalytisch aktive Zonen (5) angeordnet sind, umfassend jeweils eine Packung aus neben- und/oder übereinander gestapelten Monolithen (4) und wobei vor jeder katalytisch aktiven Zone (5) jeweils eine Mischzone (6) mit festen Einbauten vorgesehen ist,
- mit einer oder mehreren Zuführleitungen (7) für den zu dehydrierenden kohlenwasserstoffhaltigen Gasstrom (2) am unteren Ende des Reaktors (1),
- mit einer oder mehreren, unabhängig voneinander regelbaren Zuführleitungen (9), wobei jede Zuführleitung (9) einen oder mehrere Verteiler (10) versorgt, für den Sauerstoff enthaltenden Gasstrom (3) in jede der Mischzonen (6) sowie
- mit einer oder mehreren Abführleitungen (11) für das Reaktionsgasgemisch der autothermen Gasphasendehydrierung am oberen Ende des Reaktors (1)
wobei die Innenwand des Reaktors (1) durchgehend mit einer Isolationsschicht (13, 14, 15) versehen ist und wobei die Zugänglichkeit der einen oder jeder der mehreren katalytisch aktiven Zonen (5) von außerhalb des Reaktors dadurch gewährleistet ist,
- dass jeweils ein oder mehrere Mannlöcher (12) vorgesehen sind,
oder
- dass jede katalytisch aktive Zone (5) zusammen mit der zugehörigen Mischzone (6) mit festen Einbauten, den zugehörigen Sauerstoff-Zuführleitungen (9) und Verteilern (10) jeweils als ein Bauelement (24) ausgebildet ist, das einzeln montier- und demontierbar ist.

2. Reaktor (1) nach Anspruch 1, **dadurch gekennzeichnet, dass** die Bauelemente (24) mittels Flanschen (25) einzeln montier- und demontierbar sind.

3. Reaktor (1) nach einem der Ansprüche 1 oder 2, **dadurch gekennzeichnet, dass** die Isolationsschicht (13, 14, 15) im Bereich der katalytisch aktiven Zonen (5) doppellagig ausgeführt ist, mit einer ersten, an der Innenwand des Reaktors (1) anliegenden, druckstabilen Lage (13), sowie einer zweiten, zum Reaktorinnenraum hin ausgerichteten Lage (14), die aus einer Blähmatte gebildet ist, und in den übrigen Bereichen einlagig, aus einer hochtemperaturstabilen Fasermatte (15), die zum Reaktorinnenraum hin eine Blechverkleidung aufweist.

4. Reaktor (1) nach einem der Ansprüche 1 bis 3, **dadurch gekennzeichnet, dass** die Packung aus nebeneinander und übereinander gestapelten Monolithen (4) auf einem Auflagerost (16) aufliegt, wobei im unmittelbar an den Auflagerost (16) angrenzenden Bereich eine oder mehrere Lagen von Monolithen (4) vorgesehen sind, die Kanäle mit größerem Querschnitt gegenüber den übrigen, vom Auflagerost (16) weiter entfernt liegenden Monolithen (4), aufweisen.

5. Reaktor (1) nach einem der Ansprüche 1 bis 4, **dadurch gekennzeichnet, dass** im unmittelbar an den Auflagerost (16) ansetzenden Bereich eine Lage aus einer offenporigen Schaumkeramik mit einem durchströmbaren Lückenvolumen von 70 bis 90 % vorgesehen ist.

6. Reaktor (1) nach Anspruch 4 oder 5, **dadurch gekennzeichnet, dass** im unmittelbar an den Auflagerost (16) angrenzenden Bereich eine erste Lage aus einer hochporösen offenporigen Schaumkeramik, insbesondere im Bereich von 10 bis 100 mm, bevorzugt im Bereich von 40 bis 60 mm, vorgesehen ist, und darüber eine zweite Lage, die aus Monolithen (4) gebildet ist, die Kanäle mit größerem Querschnitt gegenüber den übrigen, vom Auflagerost (16) weiter entfernt liegenden Monolithen (4), aufweisen.

7. Reaktor (1) nach einem der Ansprüche 1 bis 6, **dadurch gekennzeichnet, dass** oberhalb der obersten katalytisch aktiven Zone (5) oder außerhalb des Reaktors (1) ein Wärmetauscher (17) angeordnet ist, wobei der zu dehydrierende kohlenwasserstoffhaltige Gasstrom (2) über eine Zuführleitung (7) in den Wärmetauscher (17) eingeleitet, im Wärmetauscher (17) durch das Reaktionsgasgemisch im Gegenstrom durch indirekten Wärmetausch aufgeheizt und weiter an das untere Ende des Reaktors (1) geleitet, dort über einen Stutzen (18) in den Reaktor (1) eingeleitet und in den Mischzonen (6) mit dem Sauerstoff enthaltenden Gasstrom (3) vermischt wird, worauf im Reaktor (1) die autotherme Gasphasendehydrierung stattfindet, wobei der Wärmetauscher (17) vorzugsweise mittels Flanschen (25) montier- und demontierbar ist.

8. Reaktor (1) nach einem der Ansprüche 1 bis 7, **dadurch gekennzeichnet, dass** der zu dehydrierende kohlenwasserstoffhaltige Gasstrom (2) an zwei oder mehreren Stellen in den Wärmetauscher (17) eingeleitet wird, bevorzugt als ein Hauptstrom mit höherem Massenstrom und ein oder mehrere Nebenströme mit niedrigerem Massenstrom gegenüber dem Hauptstrom.

9. Reaktor (1) nach einem der Ansprüche 7 oder 8, **dadurch gekennzeichnet, dass** zusätzlich zum Wärmetauscher (17) eine oder mehrere Zusatzheizungen für den zu dehydrierenden kohlenwasserstoffhaltigen Gasstrom (2) vorgesehen sind, wobei als Zusatzheizung vorzugsweise eine elektrische Heizung vorgesehen ist, die bevorzugt lösbar, als Einstecksystem oder als Muffelbrenner, in den zu dehydrierenden kohlenwasserstoffhaltigen Gasstrom (2) nach dem Austritt desselben aus dem Wärmetauscher (12).

10. Reaktor (1) nach einem der Ansprüche 1 bis 9, **dadurch gekennzeichnet, dass** im Reaktor (1) zwei oder mehrere katalytisch aktive Zonen (5) mit jeweils einer Packung aus nebeneinander und übereinander gestapelten Monolithen (4) vorgesehen sind.

11. Reaktor (1) nach einem der Ansprüche 1 bis 10, **dadurch gekennzeichnet, dass** die Monolithe (4) innerhalb derselben katalytisch aktiven Zone (5) mit jeweils unterschiedlicher katalytischer Aktivität ausgebildet sind.

12. Reaktor (1) nach Anspruch 10 oder 11, **dadurch gekennzeichnet, dass** die zwei oder mehreren katalytisch aktiven Zonen (5) mit jeweils unterschiedlicher katalytischer Aktivität ausgebildet sind.

13. Reaktor (1) nach einem der Ansprüche 1 bis 12, **dadurch gekennzeichnet, dass** die nebeneinander und übereinander zu einer Packung gestapelten Monolithen (4) in einer Blähmatte oder in ein Mineralfaservlies eingehüllt und in eine Einhausung mit Verspanneinrichtung eingesetzt sind.

14. Reaktor (1) nach einem der Ansprüche 1 bis 13, **dadurch gekennzeichnet, dass** jede Mischzone (6) jeweils einen Rohrverteiler umfasst, gebildet aus einer Vielzahl von parallel zueinander, in einer Ebene senkrecht zur Längsrichtung des Reaktors (1) angeordneten Einsteckrohren (19), die mit einer oder mehreren Verteilerkammern verbunden sind, und die eine Vielzahl von gleichmäßig zueinander beabstandeten Austrittsöffnungen für den Sauerstoff enthaltenden Gasstrom (3) aus den Einsteckrohren (19) aufweisen, sowie einer Vielzahl von gleichmäßig zueinander beabstandeten Mischkörpern.

15. Verfahren zur Durchführung einer autothermen Gasphasendehydrierung unter Verwendung eines Reaktors nach einem der Ansprüche 1 bis 14, wobei die autotherme Gasphasendehydrierung bevorzugt eine Dehydrierung von Propan, von Butan, von Isobutan, von Buten zu Butadien, von Ethylbenzol zu Styrol oder von Ethan zu Ethen ist.

## Claims

1. A reactor (1) in the form of a cylinder having a vertical longitudinal axis for carrying out an autothermal gas-phase dehydrogenation of a hydrocarbon-comprising gas stream (2) by means of an oxygen-comprising gas stream (3) to give a reaction gas mixture over a heterogeneous catalyst which is configured as a monolith (4), wherein
- one or more catalytically active zones (5) each comprising a packing composed of monoliths (4) stacked next to one another and/or above one another are arranged in the interior space of the reactor (1) and a mixing zone (6) having fixed internals is provided upstream of each catalytically active zone (5),
- with one or more feed lines (7) for the hydrocarbon-comprising gas stream (2) to be dehydrogenated at the lower end of the reactor (1),
- with one or more independently regulable feed lines (9), where each feed line (9) supplies one or more distributors (10), for the oxygen-comprising gas stream (3) into each of the mixing zones (6) and
- with one or more discharge lines (11) for the reaction gas mixture of the autothermal gas-phase dehydrogenation at the upper end of the reactor (1),
wherein the interior wall of the reactor (1) is provided over its entire area with an insulation layer (13,14,15) and wherein accessibility of the one or of each of the plurality of catalytically active zones (5) from outside the reactor is ensured
- by in each case one or more manholes (12) being provided or
- by each catalytically active zone (5) together with the associated mixing zone (6) having fixed internals, the associated oxygen feed lines (9) and distributors (10) being in each case configured as one module (24), fittable and removable individually.

2. The reactor (1) according to claim 1, wherein the modules (24) are individually fittable and removable by means of flanges (25).

3. The reactor (1) according to either of claims 1 and 2, wherein the insulation layer (13,14,15) is a double layer having a first pressure-stable layer (13) resting against the interior wall of the reactor (1) and a second layer (14) formed by an expandable mat facing the interior of the reactor in the region of the catalytically active zones (5) and is in the form of a single layer composed of a high-temperature-stable fiber mat (15) provided on the side facing the interior of the reactor with a sheet metal cladding in the other regions.

4. The reactor (1) according to any of claims 1 to 3, wherein the packing composed of monoliths (4) stacked next to one another and above one another rests on a support grating (16), with the region directly adjoining the support grating (16) being provided with one or more layers of monoliths (4) which have channels having a larger cross section compared to the other monoliths (4) located further away from the support grating (16).

5. The reactor (1) according to any of claims 1 to 4, wherein the region directly adjoining the support grating (16) is provided with a layer of an open-pored foam ceramic having a gap volume through which flow occurs of from 70 to 90%.

6. The reactor (1) according to claim 4 or 5, wherein the region directly adjoining the support grating (16) is provided with a first layer of a high-porosity open-pored foam ceramic, in particular in the region from 10 to 100 mm, preferably in the region from 40 to 60 mm, and a second layer formed by monoliths (4) which have channels having a larger cross section compared to the other monoliths (4) located further away from the support grating (16) is located above the first layer.

7. The reactor (1) according to any of claims 1 to 6, wherein a heat exchanger (17) is arranged above the uppermost catalytically active zone (5) or outside the reactor (1), where the hydrocarbon-comprising gas stream (2) to be dehydrogenated is introduced via a feed line (7) into the heat exchanger (17), heated by the reaction gas mixture in countercurrent by indirect heat exchange in the heat exchanger (17) and conveyed further to the lower end of the reactor (1), introduced via a port (18) into the reactor (1) and mixed with the oxygen-comprising gas stream (3) in the mixing zones (6), whereupon the autothermal gas-phase dehydrogenation takes place in the reactor (1), wherein the heat exchanger (17) is fittable and removable preferably by means of flanges (25).

8. The reactor (1) according to any of claims 1 to 7, wherein the hydrocarbon-comprising gas stream (2) to be dehydrogenated is introduced at two or more points into the heat exchanger (17), preferably as a main stream having a relatively high mass flow and one or more secondary streams having a lower mass flow than the main stream.

9. The reactor (1) according to either of claims 7 and 8, wherein one or more supplementary heating facilities are provided in addition to the heat exchanger (17) for the hydrocarbon-comprising gas stream (2) to be dehydrogenated, wherein preferably an electric heating element, which preferably detachable, as a plug-in system or as a muffle burner, is provided as supplementary heating facility in the hydrocarbon-comprising gas stream (2) to be dehydrogenated after the stream leaves the heat exchanger (12).

10. The reactor (1) according to any of claims 1 to 9, wherein two or more catalytically active zones (5) each having a packing composed of monoliths (4) stacked next to one another and above one another are provided in the reactor (1).

11. The reactor (1) according to any of claims 1 to 10, wherein the monoliths (4) within the same catalytically active zone (5) in each case have a different catalytic activity.

12. The reactor (1) according to claim 10 or 11, wherein the two or more catalytically active zones (5) in each case have a different catalytic activity.

13. The reactor (1) according to any of claims 1 to 12, wherein the monoliths (4) stacked next to one another and above one another to form a packing are enveloped in an expandable mat or in a mineral fiber nonwoven and inserted in a casing having a clamping device.

14. The reactor (1) according to any of claims 1 to 13, wherein each mixing zone (6) comprises in each case a tube distributor configured as a plurality of parallel plug-in tubes (19) which are arranged in a plane perpendicular to the longitudinal direction of the reactor (1) and are connected to one or more distribution chambers and have a plurality of uniformly spaced exit openings for the oxygen-comprising gas stream (3) from the plug-in tubes (19), and also a plurality of uniformly spaced mixing elements.

15. A process for carrying out an autothermal gas-phase dehydrogenation using a reactor according to any of claims 1 to 14, wherein the autothermal gas-phase dehydrogenation is preferably a dehydrogenation of propane, of butane, of isobutane, of butene to butadiene, of ethylbenzene to styrene or of ethane to ethene.

## Revendications

1. Réacteur (1) sous la forme d'un cylindre présentant un axe longitudinal vertical pour la réalisation d'une déshydrogénation autotherme en phase gazeuse d'un courant gazeux contenant des hydrocarbures (2) avec un courant gazeux contenant de l'oxygène (3) pour obtenir un mélange réactionnel gazeux, sur un catalyseur hétérogène, qui est configuré sous la forme d'un monolithe (4),
- une ou plusieurs zones catalytiquement actives (5) étant agencées dans l'espace intérieur du réacteur (1), comprenant chacune un garnissage de monolithes (4) empilés les uns à côté des autres et/ou les uns sur les autres, et une zone de mélange (6) comprenant des composants internes fixes étant prévue avant chaque zone catalytiquement active (5),
- avec une ou plusieurs conduites d'alimentation (7) pour le courant gazeux contenant des hydrocarbures à déshydrogéner (2) à l'extrémité inférieure du réacteur (1),
- avec une ou plusieurs conduites d'alimentation (9) réglables indépendamment les unes des autres, chaque conduite d'alimentation (9) alimentant un ou plusieurs distributeurs (10) pour le courant gazeux contenant de l'oxygène (3) dans chacune des zones de mélange (6), et
- avec une ou plusieurs conduites de déchargement (11) pour le mélange réactionnel gazeux de la déshydrogénation autotherme en phase gazeuse à l'extrémité supérieure du réacteur (1),
l'ensemble de la paroi intérieure du réacteur (1) étant munie d'une couche d'isolation (13, 14, 15) et l'accessibilité de la ou de chacune des zones catalytiquement actives (5) depuis l'extérieur du réacteur étant assurée en ce que
- un ou plusieurs trous d'homme (12) sont prévus,
ou
- chaque zone catalytiquement active (5), conjointement avec la zone de mélange (6) contenant des composants internes fixes correspondante, les conduites d'alimentation d'oxygène (9) et les distributeurs (10) correspondants, est configurée sous la forme d'une unité (24), qui peut être montée et démontée individuellement.

2. Réacteur (1) selon la revendication 1, **caractérisé en ce que** les unités (24) peuvent être montées et démontées individuellement au moyen de brides (25).

3. Réacteur (1) selon l'une quelconque des revendications 1 ou 2, **caractérisé en ce que** la couche d'isolation (13, 14, 15) est configurée à double couche dans la partie des zones catalytiquement actives (5), avec une première couche (13) stable à la pression, adjacente à la paroi intérieure du réacteur (1), et une deuxième couche (14) orientée vers l'espace intérieur du réacteur, qui est formée par un mat gonflé, et à une couche dans les autres parties, en un mat de fibres stable aux températures élevées (15), qui comprend un revêtement de tôle du côté de l'espace intérieur du réacteur.

4. Réacteur (1) selon l'une quelconque des revendications 1 à 3, **caractérisé en ce que** le garnissage de monolithes (4) empilés les uns à côté des autres et les uns sur les autres est disposé sur une grille support (16), une ou plusieurs couches de monolithes (4) étant prévues dans la zone directement adjacente à la grille support (16), qui présentent des canaux de section transversale plus grande que les autres monolithes (4) plus éloignés de la grille support (16).

5. Réacteur (1) selon l'une quelconque des revendications 1 à 4, **caractérisé en ce qu'**une couche d'une céramique moussée à pores ouverts ayant un volume d'espaces traversables de 70 à 90 % est prévue dans la zone directement adjacente à la grille support (16).

6. Réacteur (1) selon la revendication 4 ou 5, **caractérisé en ce qu'**une première couche de céramique moussée à pores ouverts hautement poreuse est prévue dans la zone directement adjacente à la grille support (16), notamment dans la zone de 10 à 100 mm, de préférence dans la zone de 40 à 60 mm, et sur celle-ci une deuxième couche, qui est formée par des monolithes (4) qui présentent des canaux ayant une section transversale plus grande que les autres monolithes (4) plus éloignés de la grille support (16).

7. Réacteur (1) selon l'une quelconque des revendications 1 à 6, **caractérisé en ce qu'**un échangeur de chaleur (17) est agencé au-dessus de la zone catalytiquement active la plus supérieure (5) ou à l'extérieur du réacteur (1), le courant gazeux contenant des hydrocarbures à déshydrogéner (2) étant introduit par une conduite d'alimentation (7) dans l'échangeur de chaleur (17), chauffé par échange de chaleur indirect dans l'échangeur de chaleur (17) par le mélange réactionnel gazeux à contre-courant, puis conduit à l'extrémité inférieure du réacteur (1), y étant introduit dans le réacteur (1) par un raccord (18) et mélangé dans les zones de mélange (6) avec le courant gazeux contenant de l'oxygène (3), la déshydrogénation autotherme en phase gazeuse ayant alors lieu dans le réacteur (1), l'échangeur de chaleur (17) pouvant de préférence être monté et démonté au moyen de brides (25).

8. Réacteur (1) selon l'une quelconque des revendications 1 à 7, **caractérisé en ce que** le courant gazeux contenant des hydrocarbures à déshydrogéner (2) est introduit dans l'échangeur de chaleur (17) à deux emplacements ou plus, de préférence sous la forme d'un courant principal de débit massique plus élevé et d'un ou de plusieurs courants secondaires de débit massique plus faible par rapport au courant principal.

9. Réacteur (1) selon l'une quelconque des revendications 7 ou 8, **caractérisé en ce qu'**un ou plusieurs dispositifs de chauffage supplémentaires sont prévus pour le courant gazeux contenant des hydrocarbures à déshydrogéner (2) en plus de l'échangeur de chaleur (17), un dispositif de chauffage électrique étant de préférence prévu en tant que dispositif de chauffage supplémentaire, qui de préférence amovible, sous la forme d'un système à insérer ou sous la forme d'un brûleur à moufle, dans le courant gazeux contenant des hydrocarbures à déshydrogéner (2) après la sortie de celui-ci de l'échangeur de chaleur (12).

10. Réacteur (1) selon l'une quelconque des revendications 1 à 9, **caractérisé en ce que** deux zones catalytiquement actives (5) ou plus, contenant chacune un garnissage de monolithes (4) empilés les uns à côté des autres et les uns sur les autres, sont prévues dans le réacteur (1).

11. Réacteur (1) selon l'une quelconque des revendications 1 à 10, **caractérisé en ce que** les monolithes (4) sont configurés dans une même zone catalytiquement active (5) avec chacun une activité catalytique différente.

12. Réacteur (1) selon la revendication 10 ou 11, **caractérisé en ce que** les deux zones catalytiquement actives (5) ou plus sont configurées avec chacune une activité catalytique différente.

13. Réacteur (1) selon l'une quelconque des revendications 1 à 12, **caractérisé en ce que** les monolithes (4) empilés les uns à côté des autres et les uns sur les autres en un garnissage sont gainés dans un mat gonflé ou dans un non-tissé de fibres minérales, et utilisés dans une enceinte munie d'un dispositif de serrage.

14. Réacteur (1) selon l'une quelconque des revendications 1 à 13, **caractérisé en ce que** chaque zone de mélange (6) comprend un distributeur à tubes, formé par une pluralité de tubes insérables (19) agencés parallèlement les uns aux autres, dans un plan perpendiculaire à la direction longitudinale du réacteur (1), qui sont reliés avec une ou plusieurs chambres de distribution, et qui comprennent une pluralité d'ouvertures de sortie espacées uniformément les unes des autres pour le courant gazeux contenant de l'oxygène (3) issu des tubes insérables (19), ainsi qu'une pluralité de corps de mélange espacés uniformément les uns des autres.

15. Procédé de réalisation d'une déshydrogénation autotherme en phase gazeuse utilisant un réacteur selon l'une quelconque des revendications 1 à 14, la déshydrogénation autotherme en phase gazeuse étant de préférence une déshydrogénation de propane, de butane, d'isobutane, de butène en butadiène, d'éthylbenzène en styrène ou d'éthane en éthène.
